# DEMANDE DE BREVET EUROPEEN

(11) **EP 0 576 347 A1**
(43) Date de publication de la demande: **29.12.1993**
(21) Numéro de dépôt: 93401600.7
(22) Date de dépôt: 22.06.1993
(51) Int. Cl.: C07D 209/30

(54) **Dérivés hydroxy-4 benzènethio, leur préparation ainsi que leur utilisation pour la préparation de dérivés aminoalkoxybenzènesulfonyles**

(30) Priorité: 23.06.1992 FR 9207659
(71) Demandeur: SANOFI, F-75008 Paris (FR)
(72) Inventeur: Gubin, Jean, B-1020 Bruxelles (BE); Inion, Henri, B-1780 Wemmel (BE)
(74) Mandataire: Le Guen, Gérard

(57) **Abrégé**

L'invention a pour objet des dérivés hydroxy-4 benzènethio de formule générale : 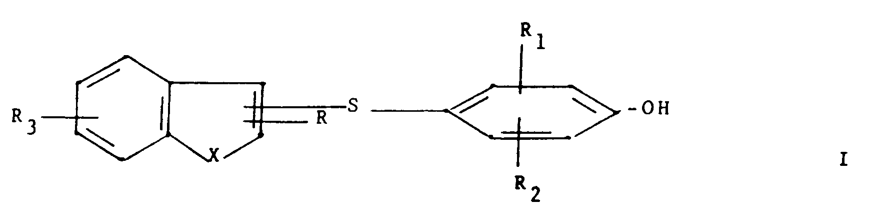
dans laquelle :
R₁ et R₂, identiques ou différents, représentent chacun l'hydrogène, le radical méthyle ou éthyle ou un atome d'halogène,
R représente un radical alkyle en C₁-C₆, cycloalkyle en C₃-C₆ ou phényle,
R₃ représente l'hydrogène ou un atome d'halogène,
X représente -O-, -S- ou 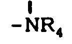
dans lequel R₄ représente l'hydrogène ou un groupement alkyle en C₁-C₄,utiles comme intermédiaires de synthèse, notamment pour la préparation de dérivés aminoalkoxybenzènesulfonyles qui sont des composés pharmaceutiquement actifs.

## Description

La présente invention se rapporte, d'une manière générale, à de nouveaux dérivés de thiophénol, à leur procédé de préparation ainsi qu'à leur utilisation comme intermédiaires de synthèse.

Plus précisément, l'invention a pour objet les dérivés hydroxy-4 benzènethio de formule générale: 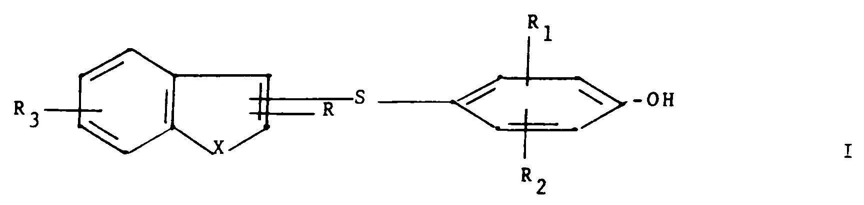
dans laquelle :
R₁' et R₂ identiques ou différents, représentent chacun l'hydrogène, le radical méthyle ou éthyle ou un atome d'halogène tel que chlore, brome ou iode,
R représente un radical alkyle en C₁-C₆, cycloalkyle en C₃-C₆ ou phényle,
R₃ représente l'hydrogène ou un atome d'halogène tel que chlore ou brome,
X représente -O-, -S- ou 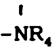
dans lequel R₄ représente l'hydrogène ou un groupement alkyle en C₁-C₄.

Ainsi, en tenant compte des valeurs ci-dessus , R peut représenter notammment le radical méthyle, éthyle, n-propyle, isopropyle, n-butyle, n-pentyle, n-hexyle ou cyclopropyle.

Les composés de formule I dans laquelle R₁ et R₂ représentent chacun l'hydrogène, R₃ représente l'hydrogène ou un atome de chlore et X représente un radical -NR₄ dans lequel R₄ représente l'hydrogène ou le radical méthyle, constituent des composés préférés selon l'invention.

De même, une série particulièrement préférée de composés est représentée par la formule I dans laquelle X représente un groupement

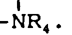

Dans la formule I ci-dessus, le groupement hydroxy-4 benzènethio peut se trouver en position 2 de l'hétérocycle et le groupement R en position 3, ou inversement, de manière à former des dérivés (R)-3 (hydroxy-4 benzènethio)-2 benzofuranne, benzothiophène ou indole ou des dérivés (R)-2 (hydroxy-4 benzènethio)-3 benzofuranne, benzothiophène ou indole. Toutefois, on préfère les dérivés (R)-3 (hydroxy-4 benzènethio)-2 indole de formule I.

Les composés de l'invention se sont révélés particulièrement utiles comme produits intermédiaires, notamment pour la préparation des dérivés hydroxy-4 benzènesulfonyles de formule générale : 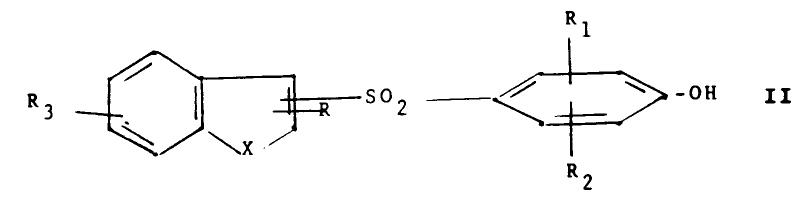
dans laquelle R, R₁, R₂, R₃ et X ont la même signification que précédemment.

Ces composés de formule II peuvent être eux-mêmes largement utilisés comme intermédiaires dans la préparation de différents produits, notamment pour la synthèse finale de dérivés aminoalkoxybenzènesulfonyles de formule générale : 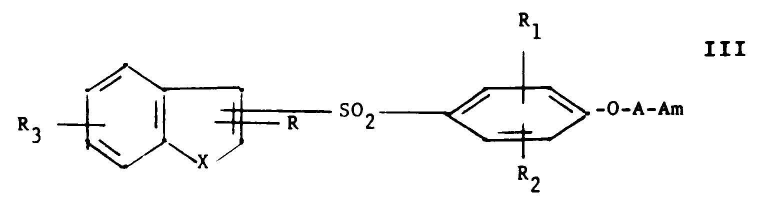
dans laquelle R, R₁, R₂, R₃ et X ont la même signification que précédemment, A représente un radical alkylène en C₂-C₅ ou hydroxy-2 propylène, et Am représente un radical amino substitué, notamment :
- un radical de formule : 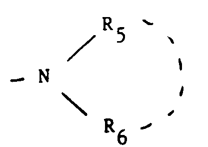
dans laquelle R₅ représente l'hydrogène ou un radical alkyle en C₁ - C₈ et R₆ représente un radical alkyle en C₁ - C₈ ou un radical de formule

- Alk - R₇

dans laquelle Alk représente une liaison simple ou un radical alkylène en C₁ - C₅ et R₇ représente un radical pyridyle, phényle, méthylènedioxy -2,3 phényle, méthylènedioxy -3,4 phényle ou un groupement phényle substitué par un ou plusieurs substituants identiques ou différents sélectionnés parmi des atomes d'halogène, des groupements alkyles en C₁-C₄ ou des groupements alkoxy en C₁-C₄ ou R₅ et R₆, lorsqu'ils sont pris ensemble, représentent un radical alkylène ou alkénylène en C₃-C₆, éventuellement interrompu par -O-, -NH-, -N= ou 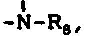
R₈ représentant un radical alkyle en C₁-C₄, phényle ou diphénylméthyle, de sorte que R₅ et R₆, pris avec l'atome d'azote auxquels ils sont attachés, peuvent représenter notamment un radical pyrrolidinyle, pipéridinyle, morpholinyle, pipérazinyle, méthyl-4 pipérazinyle,phényl-4 pipérazinyle ou 1H-imidazolyle,
un group**e** de formule : 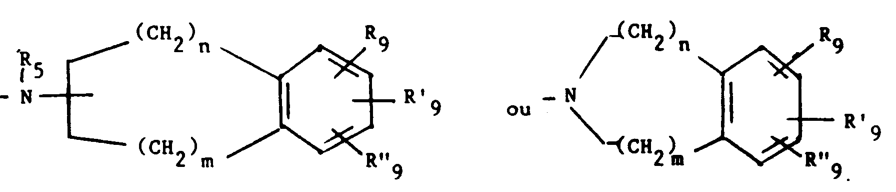
dans lesquelles R₅ a la même signification que précédemment, R₉, R'₉ et R''₉, qui sont identiques ou différents, représentent chacun l'hydrogène, un atome d'halogène tel que chlore ou brome, un groupement alkyle en C₁-C₄ ou alkoxy en C₁-C₄ et n et m, identiques ou différents, représentent chacun 0, 1, 2 ou 3,
ainsi que pour la préparation de leurs sels pharmaceutiquement acceptables.

De tels dérivés aminoalkoxybenzènesulfonyles de formule III ont été décrits notamment dans les brevets ou demandes de brevet EP-A-0302 793, 0382618, 0382628 et 0382629.

Ces composés se sont révélés particulièrement intéressants pour leurs applications thérapeutiques notamment pour leurs propriétés inhibitrices de la translocation calcique ainsi que leurs propriétés bradycardisantes, hypotensives ou antiadrénergiques qui les rendent utiles dans le traitement de certains syndromes pathologiques du sytème cardiovasculaire, en particulier dans le traitement de l'angine de poitrine, de l'hypertension, de l'arythmie, de l'insuffisance circulatoire cérébrale. Dans le domaine antitumoral, ces composés pourront être utiles comme potentialisateurs d'anticancéreux.

Comme composés particulièrement représentatifs de cette série de dérivés aminoalkoxybenzènesulfonyles, on peut citer :
- le méthyl-1 isopropyl-3 [{[N-méthyl N-(diméthoxy-3,4 β- phénéthyl) amino]-3 propoxy}-4 benzènesulfonyl]-2 indole et ses sels pharmaceutiquement acceptables (Composé A).
- le méthyl-1 isopropyl-3 [{[N-méthyl N-(diméthoxy-3,5 β-phénéthyl) amino]-3 propoxy}-4 benzèsulfony]-2 indole et ses sels pharmaceutiquement acceptables (Composé B).
- le [{[N-méthyl N-(diméthoxy-3,4 β-phénéthyl) amino]-3 propoxy}-4 benzènesulfony]-2 chloro-5 isopropyl-3 méthyl-1 indole et ses sels pharmaceutiquement acceptables (Composé C).

On a rapporté dans les brevets cités précédemment, un procédé pour la préparation de dérivés hétérocycliques aminoalkoxybenzènesulfonyles au départ de dérivés hétérocycliques hydroxy-4 benzènesulfonyles, notamment des dérivés hydroxy-4 benzènesulfonyles de benzofuranne, benzothiophène ou indole. En outre, on y a cité une méthode pour la synthèse de ces dérivés hydroxy-4 benzènesulfonyles. Celle-ci comporte notamment une réaction d'oxydation d'un dérivé méthoxy-4 benzènethio hétérocyclique, au moyen d'acide chloro-3 perbenzoïque.

La demande de brevet EP-A-0302793 décrit par exemple la synthèse en 4 étapes de l'isopropyl-3 méthyl-1 (hydroxy-4 benzènesulfonyl)-2 indole au départ d'isopropyl-3 indole selon un procédé faisant appel à une réaction d'oxydation de ce type.

Ce procédé consiste à :
a) condenser l'isopropyl-3 indole avec le méthoxy-4 benzènethiol pour donner l'isopropyl-3 (méthoxy-4 benzènethio)-2 indole (rendement : 72% en produit pur);
b) oxyder ce composé avec l'acide chloro-3 perbenzoique pour donner l'isopropyl-3 (méthoxy-4 benzènesulfonyl)-2 indole (rendement : 90%) ;
c) méthyler le composé obtenu au moyen d'iodure de méthyle en présence d'hydrure de sodium, ce qui fournit le méthyl-1 isopropyl-3 (méthoxy-4 benzènesulfonyl)-2 indole (rendement : 85%) ;
d) O- déprotéger ce composé au moyen de mercapto-2 éthanol/hydrure de sodium pour obtenir finalement le composé souhaité (rendement : 45,9 %).

En conséquence, l'isopropyl-3 méthyl-1 (hydroxy-4 benzènesulfonyl)-2 indole peut être synthétisé selon ce procédé, avec un rendement global faible puisque d'environ 28% à partir d'isopropyl-3 indole.

En outre, l'utilisation de mercapto-2 éthanol laisse une odeur nauséabonde au sein du dérivé hydroxy-4 benzènesulfonyle de formule II, odeur qui se retrouve dans le dérivé aminoalkoxybenzènesulfonyle final de formule III.

La méthode ainsi décrite se caractérise notamment par la mise en oeuvre d'une étape d'oxydation d'un dérivé benzènethio comportant un radical hydroxyle protégé, en l'occurence un radical méthoxy, radical hydroxyle protégé qu'il est nécéssaire de déprotéger dans la suite du procédé, de manière à régénérer l'hydroxyle libre.

Cette mise en oeuvre selon un couple oxydation / déprotection peut s'expliquer par le fait que la fonction hydroxyle libre est bien connue pour être sensible aux agents d'oxydation puisque capable de s'oxyder assez facilement [Methoden der Organischen Chemie (Houben-Weyl), Band VI/1c - Phenole Teil 2, page 1121].

La recherche d'un procédé industriel pour la préparation de dérivés hydroxy-4 benzènesulfonyles hétérocycliques de formule II mettant en oeuvre des intermédiaires de synthèse d'accès facile et peu onéreux avec un rendement satisfaisant en produit final reste d'un intérêt inconstestable.

Or, on a découvert de manière surprenante, selon l'invention, qu'il est possible d'obtenir avec d'excellents rendements des dérivés hydroxy-4 benzènesulfonyles hétérocycliques, notamment des dérivés d'(hydroxy-4 benzènesulfonyl)-2 ou -3 indole, par oxydation de dérivés benzènethio hétérocycliques comportant non pas un radical hydroxyle protégé mais un radical hydroxyle libre.

Selon l'invention, on prépare les composés de formule II en oxydant un dérivé benzènethio de formule I au moyen d'un agent d'oxydation approprié tel que par exemple l'acide chloro-3 perbenzoique ou le monoperphtalate de magnésium et dans un solvant approprié, ce qui fournit les composés désirés.

Généralement, l'oxydation se déroule à une température comprise entre -5°C et la température ambiante, de préférence à une température comprise entre 0°C et la température ambiante.

Quand au solvant, il peut s'agir d'un solvant polaire comportant un groupement amido par exemple le N, N- diméthylformamide, le N, N-diméthylacétamide, la méthyl-2 pyrrolidone, ou l'hexaméthylphosphotriamide, un alcool inférieur par exemple, le méthanol ou l'éthanol ou encore un nitrile tel que l'acétonitrile.

Le N, N-diméthylformamide constitue un solvant particulièrement préféré.

En général, on utilise de 2 à 2,5 équivalents d'agent oxydant, de préférence d'acide chloro-3 perbenzoïque ou de monoperphtalate de magnésium, par équivalent de composé de formule I.

En outre, on peut envisager de tamponner le milieu réactionnel par introduction d'une base faible telle qu'un carbonate ou bicarbonate de métal alcalin ou alcalino-terreux.

Les composés de formule I peuvent donner accès aux composés de formule II de manière très avantageuse tout en évitant les inconvénients des procédés antérieurs.

En effet, la formation d'une chaine hydroxy-4 benzènesulfonyle à partir des dérivés hydroxy-4 benzènethio de formule I peut être réalisée au moyen d'une réaction unique au contraire des procédés antérieurs qui nécessitent la mise en oeuvre d'une double réaction c'est-à-dire une oxydation d'abord, une déprotection du radical hydroxyle ensuite.

En outre, les composés de formule I permettent la synthèse de composés de formule II avec des rendements particulièrement intéressants puisque largement supérieurs à ceux obtenus selon la technique antérieure.

Par exemple, le méthyl-1 isopropyl-3 (hydroxy-4 benzènesulfonyl)-2 indole peut être préparé à partir d'isopropyl-3 indole via l'(hydroxy-4 benzènethio)-2 méthyl-1 isopropyl-3 indole par mise en oeuvre de trois étapes contrairement à la technique antérieure qui nécessite un ensemble de quatre étapes. Au surplus, les rendements globaux obtenus selon l'invention s'avèrent supérieurs à ceux fournis par les procédés connus puisque de l'ordre de 65% à 70% à partir de l'isopropyl-3 indole.

En conséquence, un autre objet de l'invention concerne les dérivés hydroxy-4 benzènethio de formule I, en tant que produits industriels nouveaux, utiles notamment comme intermédiaires de synthèse, par exemple pour la préparation des dérivés hydroxy-4 benzènesulfonyles de formule II.

Les composés de formule I peuvent être obtenus en faisant réagir un composé de formule générale: 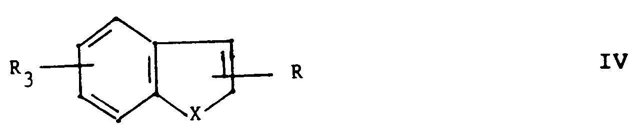
dans laquelle R, R₃ et X ont la même signification que précédemment avec un thiophènol de formule générale : 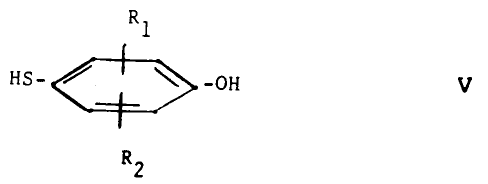
dans laquelle R₁ et R₂ ont la même signification que précédemment et ce, dans un solvant approprié tel qu'un solvant aqueux contenant un alcool en C₁-C₄, par exemple l'éthanol ou un amide tel que le N, N-diméthylformamide ou l'hexaméthylphosphotriamide, en présence d'iode et de préférence à la température de reflux du milieu réactionnel, pour obtenir les composants désirés.

Les composés de formule IV et V sont des composés connus pouvant être obtenus selon des méthodes connues.

Par exemple, les composés de formule IV dans laquelle X représente un radical -NR₄, dans lequel R₄ est autre que l'hydrogène, peuvent être obtenus en traitant un composé de formule IV dans laquelle X représente -NH, éventuellement sous forme de dérivé métallique, avec un halogénure de formule générale :

Hal-R₄ VI

dans laquelle Hal représente un atome d'halogène, par exemple d'iode et R₄ a la même signification que précédemment à l'exception d'hydrogène.

Préférentiellement, on utilise un dérivé métallique du composé de formule IV dans laquelle X représente 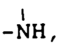
dérivé métallique obtenu en traitant le composé de formule IV en question dans laquelle X représente 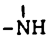
avec un hydrure ou un alcoolate de métal alcalin tel que l'hydrure de sodium ou le tertiobutylate de potassium.

Comme indiqué précédemment, les dérivés hydroxy-4 benzènethio de formule I peuvent être utilisés pour la préparation des dérivés aminoalkoxybenzènesulfonyles de formule III.

En conséquence, un autre objet de l'invention concerne les dérivés hydroxy-4 benzènethio de formule I en tant qu'intermédiaires pour la synthèse finale des dérivé aminoalkoxybenzènesulfonyles de formule III en particulier pour la synthèse des Composés A, B ou C.

Par exemple, on peut préparer les composés de formule III au départ d'un dérivé hydroxy-4 benzènesulfonyle de formule II lui-même obtenu selon l'invention à partir d'un dérivé hydroxy-4 benzènethio de formule I, par mise en oeuvre d'un procédé comportant la suite d'étapes suivantes :
a) condensation du composé de formule II, en présence d'un agent basique avec un dihalogénoalkane de formule générale :

   Hal - A - Hal VII

   dans laquelle A a la même signification que précédemment et Hal représente un atome d'halogène, de préférence brome et ce, au reflux, dans un solvant approprié, généralement un solvant polaire ou apolaire tel que la méthyl-éthyl-cétone, le N,N- diméthylformamide, le benzène, le toluène ou un xylène,
   ou bien
b₁) la condensation d'un alcool halogéné de formule générale :

   Hal - A -OH VIII

   dans laquelle A et Hal ont la même signification que précédemment et ce, dans un solvant tel que le N, N-diméthylformamide et en présence d'un agent basique, puis condensation du dérivé alcool obtenu avec un halogénure de formule générale :

   Hal - W IX

   dans laquelle Hal a la même signification que précédemment et W représente un radical alkylsulfonyle en C₁-C₄, par exemple méthanesulfonyle ou arylsulfonyle en C₆-C₁₀, par exemple benzènesulfonyle ou p-toluènesulfonyle, dans un solvant accepteur d'acide, par exemple la pyridine,
   ou bien
b₂) le chauffage au reflux avec une épihalohydrine telle que l'épichlorhydrine, en présence d'un agent basique, et dans un solvant polaire tel que la méthyl-éthyl-cétone,
   pour obtenir les dérivés sulfonyles hétérocycliques de formule générale : 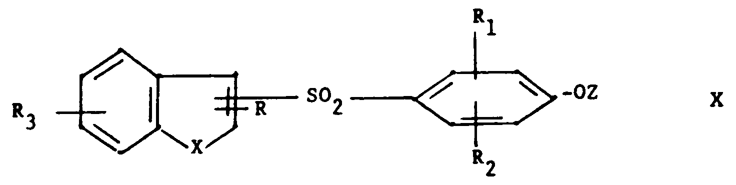 dans laquelle R, R₁, R₂, R₃ et X ont la même signification que précédemment et Z représente un radical de formule générale :

   - A - Z₁

   dans laquelle A a la même signification que précédemment et Z₁ représente un atome d'halogène, un radical alkysulfonyloxy en C₁-C₄ ou arylsulfonyloxy en C₆-C₁₀.

L'agent basique utilisé lors du traitement du composé de formmule II est généralement un carbonate de métal alcalin, par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin, par exemple, le méthylate ou l'éthylate de sodium.

On fait alors réagir le dérivé de formule X avec une amine de formule générale :

H - Am XI

dans laquelle Am a la même signification que précédemment, la réaction ayant lieu en présence d'un accepteur d'acide et dans un solvant approprié, généralement un solvant polaire tel qu'un alcool, par exemple le butanol, une,cétone telle que la méthyl-éthyl-cétone, un hydrocarbure aromatique par exemple le benzène, le toluène ou un xylène ou encore un excès d'amine de formule XI pour obtenir les composés de formule III sous forme de base libre, que l'on peut faire réagir, si on le désire, avec un acide approprié pour former un sel pharmaceutiquement acceptable de ce composé.

Suivant une méthode alternative, on peut mettre en oeuvre les composés de formule II obtenus selon l'invention en traitant directement un tel composé, avec un halogénure de formule générale :

Hal - A - Am XII

dans laquelle Hal et Am ont la même signification que précédemment et A représente un radical alkylène en C₂-C₅, la réaction étant conduite en présence d'un agent basique tel qu'un carbonate de métal alcalin, par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin, par exemple le méthylate ou l'éthylate de sodium, pour obtenir les composés de formule III dans laquelle A représente un radical alkylène C₂ -C₅, que l'on peut, si on le désire, faire réagir avec un acide approprié pour former un sel pharmaceutiquement acceptable de ce composé.

Les Exemples non limitatifs suivants illustrent la préparation des composés de l'invention :

### Exemple 1

**Préparation de l'(hydroxy-4 benzènethio)-2 isopropyl-3** **méthyl-1 indole**
a) Isopropyl-3 méthyl-1 indole
   Sous bonne agitation, on ajoute une solution de 25 g (0,157 mole) d'isopropyl-3 indole dans 200 ml de benzène à une suspension de 21,1 g (0,172 mole) de tertiobutylate de potassium.
   On ajoute ensuite lentement 24,51 g (0,172 mole) d'iodure de méthyle. On poursuit l'agitation à la température ambiante pendant 20 h.
   On ajoute un mélange eau/glace pilée et on sépare la fraction benzènique. On lave cette fraction deux fois avec de l'eau, sèche sur sulfate de sodium anhydre, filtre et chasse le solvant à l'évaporateur rotatif, ce qui fournit un résidu huileux.
   De cette manière, on obtient 27 g d'isopropyl-3 méthyl-1 indole.
   Rendement : 100%.
b) (Hydroxy-4 benzènethio)-2 isopropyl-3 méthyl-1 indole
   On dissout 27 g (0,155 mole) d'isopropyl-3 méthyl-1 indole et 21,7 g (0,172 mole) d'hydroxy-4 benzènethiol dans 250 ml d'éthanol et 125 ml d'eau. On place l'ensemble sous atmosphère d'azote et on ajoute lentement une solution de 43,65 g (0,172 mole) d'iode dans l'éthanol. On chauffe au reflux durant 2 heures, refroidit et verse dans de l'eau glacée. On extrait à l'acétate d'éthyle, lave à l'eau et sèche sur sulfate de sodium anhydre. On filtre alors, évapore le solvant à l'évaporateur rotatif et purifie par chromatographie sur colonne (éluant : heptane/dichloréthane 7/3).

De cette manière, on obtient 33,9 g d'(hydroxy-4 benzènethio)-2 isopropyl-3 méthyl-1 indole.
Rendement : 73,5 %
P.F. : 89-90°C.
Pureté : 99,3 %.

### Exemple 2

**Préparation de l'(hydroxy-4-benzenethio)-3** **isopropyl-2** **indole.**

On dissout 3,2 g (0,02 mole) d'isopropyl-2 indole et 2,5 g (0,02 mole) d'hydroxy-4 benzènethiol dans 80 ml d'éthanol.

On ajoute 40 ml d'eau, agite puis introduit, goutte-à goutte une solution de 5g (0,04 mole) d'iode dans l'éthanol. On verse alors dans 400 ml d'eau l'huile ainsi formée puis on agite tout en refroidissant jusqu'à solidification. On filtre et lave le produit sur filtre. On sèche alors sous vide à la température de 60°C ce qui fournit 5,6 g du produit attendu. On recristallise ensuite dans 250 ml d'un mélange heptane/toluène 7/3.

De cette manière, on obtient 4,2 g d'(hydroxy-4 benzènethio)-3 isopropyl-2 indole sous forme d'une résine insoluble.
Rendement : 74,2%
P.F. : 116°C.

Les Exemples non limitatifs suivants illustrent l'utilisation des composés de l'invention :

### Exemple I

**Préparation de l'(hydroxy-4 benzènesulfonyl)-2 isopropyl-****3 méthyl-1 indole**

On dissout 13,17 g (0,044 mole) d'(hydroxy-4 benzènethio)-2 isopropyl-3 méthyl-1 indole dans 70 ml de N,N-diméthylformamide. On agite et refroidit à 0°C puis on ajoute, lentement (2 h) une solution de 21,7 g (0,088 mole) d'acide chloro-3 perbenzoïque à 70%. On laisse la température du milieu remonter jusqu'à la température ambiante et on poursuit l'agitation du milieu réactionnel pendant 20 h tout en suivant l'évolution de la réaction (sulfure, sulfoxyde, sulfone) par C.C.M. On coule le mélange dans l'eau glacée, filtre le précipité formé et lave sur filtre avec une solution de bicarbonate de sodium en terminant par de l'eau. On purifie alors par chromatographie sur colonne de silice (éluant : dichloréthane contenant 2% de méthanol).

De cette manière, on obtient 9 g d'(hydroxy-4 benzènesulfonyl)-2 isopropyl-3 méthyl-1 indole.
Rendement : 62,1%.
P.F. : 188°C

### Exemple II

**Préparation de l'(hydroxy-4 benzènesulfonyl)-2 isopropyl** **-3 méthyl-1 indole**

En utilisant le même procédé que celui décrit à l'Exemple I mais en ajoutant en 2 h à température ambiante, 2,1 moles d'acide chloro-3 perbenzoïque à 1 mole d'(hydroxy-4 benzènethio)-2 isopropyl-3 méthyl-1 indole dans le N, N-diméthylformamide et en maintenant le milieu réactionnel sous agitation durant 24 h à température ambiante, on obtient l'(hydroxy-4 benzènesulfonyl)-2 isopropyl-3 méthyl-1 indole avec un rendement de 62%.

### Exemple III

**Préparation de l'(hydroxy-4 benzènesulfonyl)-2 isopro****pyl-3** **méthyl-1 indole**
En utilisant le même procédé que celui décrit à l'Exemple I, mais en ajoutant, en 5 h à 10°C, 2,4 moles d'acide chloro-3 perbenzoïque à 1 mole d'(hydroxy-4 benzènethio)-2 isopropyl-3 méthyl-1 indole dans le N, N-diméthylformamide, et en maintenant le milieu réactionnel sous agitation durant 48 h à température ambiante, on obtient l'(hydroxy-4 benzènesulfonyl)-2 isopropyl-3 méthyl-1 indole, avec un rendement de 80%.

### Exemple IV

**Préparation de l'(hydroxy-4 benzènesulfonyl)-2 isopro****pyl-3 méthyl-1 indole**

En utilisant le même procédé que celui décrit à l'Exemple I, mais en ajoutant 2,4 moles d'acide chloro-3 perbenzoïque à 1 mole d'(hydroxy-4 benzènethio)-2 isopropyl-3 méthyl-1 indole dans le N,N-diméthylformamide, à raison de 1,1 mole en 0,5h, à 10°C, puis 1,3 mole en 5h, à 10°C, et en maintenant le milieu réactionnel sous agitation durant 48 h à température ambiante, on obtient l'(hydroxy-4 benzènesulfonyl)-2 isopropyl-3 méthyl-1 indole, avec un rendement de 88%.

### Exemple V

**Préparation de l'oxalate acide de méthyl-1 isopropyl-3** **[{**[**N-méthyl N-(diméthoxy-3,4 β-phénéthyl)amino]-3 pro****poxy}-4 benzènesulfonyl]-2 indole**
A un mélange de 9,9 g (0,003 mole) d'(hydroxy-4 benzènesulfonyl)-2 isopropyl-3 méthyl-1 indole dans 165 ml de N, N-diméthylformamide, on ajoute 29 g (0,21 mole) de carbonate de potassium anhydre et finement broyé. On agite durant 0,5 heure et on ajoute 13,3 g (0,033 mole) d'oxalate acide de chloro-1 [N-méthyl N-(diméthoxy-3,4 β- phénéthyl)amino]-3 propyle à 90%.

On chauffe à 100°C pendant 1 heure et on laisse revenir à température ambiante, sous agitation. On verse dans 450 ml d'eau et de glace, et on agite durant 0,25 heure. On extrait avec 3 fois 150 ml d'éther diéthylique, lave deux fois avec 150 ml d'eau, et on sèche sur sulfate de sodium. A la solution éthérée obtenue, on ajoute 3,5 g d'acide oxalique dissous dans 50 ml d'éther éthylique, et on laisse cristalliser pendant 24 heures. On essore, lave à l'éther isopropylique et sèche sous vide à 50°C.

De cette manière, on obtient l'oxalate acide de méthyl-1 isopropyl-3 [{[N-méthyl N-(diméthoxy-3,4 β-phénéthyl)amino]-3 propoxy}-4 benzènesulfonyl]-2 indole.
Rendement : 90%
P.F. : 94°C.

### Exemple VI

**Préparation de l'(hydroxy-4 benzènesulfonyl)-3 isopro****pyl-2 indole**
On dissout 0,36 g (0,00127 mole) d'(hydroxy-4 benzènethio)-3 isopropyl-2 indole dans 5 ml de N, N-diméthyformamide. On refroidit à 0°C (bain de glace) et on ajoute, goutte à goutte, une solution de 0,62 g (0,00254 mole) d'acide chloro-3 perbenzoïque à 70% dans 2 ml de N,N-diméthylformamide.

On poursuit l'agitation à la température ambiante pendant 24 heures puis on coule dans de l'eau glacée.

On extrait à l'acétate d'éthyle le précipité collant ainsi formé et on lave la solution avec une solution aqueuse de bicarbonate de sodium puis avec de l'eau. On sèche sur sulfate de sodium, filtre et chasse le solvant à l'évaporateur rotatif, ce qui fournit un résidu de 0,5 g que l'on cristallise dans un mélange éthanol/eau.

De cette manière, on obtient 0,38 g d'(hydroxy-4 benzènesulfonyl)-3 isopropyl-2 indole.
Rendement : 95%.
P.F. : 152°C.

## Revendications

1. Dérivés hydroxy-4 benzènethio de formule générale : 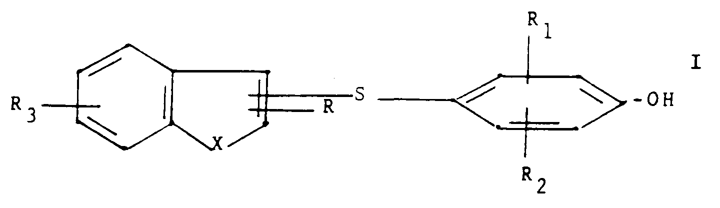 dans laquelle :
R₁ et R₂, identiques ou différents, représentent chacun l'hydrogène, le radical méthyle ou éthyle ou un atome d'halogène,
R représente un radical alkyle en C₁-C₆, cycloalkyle en C₃-C₆ ou phényle,
R₃ représente l'hydrogène ou un atome d'halogène,
X représente -O-, -S- ou 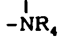 dans lequel R₄ représente l'hydrogène ou un groupement alkyle en C₁-C₄.

2. Dérivés hydroxy-4 benzènethio selon la revendication 1 dans laquelle R₁ et R₂ représentent chacun l'hydrogène, R₃ représente l'hydrogène ou un atome de chlore et X représente un radical 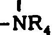 dans lequel R₄ représente l'hydrogène ou le radical méthyle.

3. Dérivés hydroxy-4 benzènethio selon la revendication 1 ou 2, caractérisés en ce qu'ils correspondent à la formule générale : 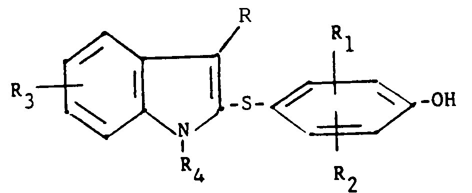 dans laquelle R,R₁, R₂, R₃ et R₄ ont la même signification que dans la revendication 1.

4. Dérivés hydroxy-4 benzènethio selon l'une des revendications 1 ou 2, caractérisés en ce que X représente un radical

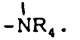

5. (Hydroxy-4 benzènethio)-2 isopropyl-3 méthyl-1 indole.

6. (Hydroxy-4 benzènethio)-3 isopropyl-2 indole.

7. Procédé de préparation de dérivés hydroxy-4 benzènethio selon la revendication 1, caractérisé en ce que l'on fait réagir un composé de formule générale: 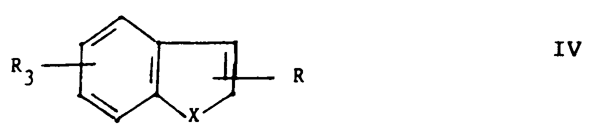 dans laquelle R, R₃, et X ont la même signification que dans la revendication 1, avec un thiophénol de formule générale : 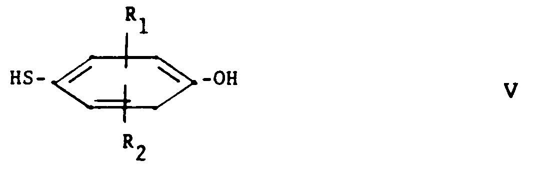 dans laquelle R₁ et R₂ ont la même signification que dans la revendication 1 et ce, dans un solvant en présence d'iode et à la température de reflux du milieu réactionnel, pour obtenir les composés désirés.

8. Procédé de préparation de dérivés hydroxy-4 benzènesulfonyles de formule générale : 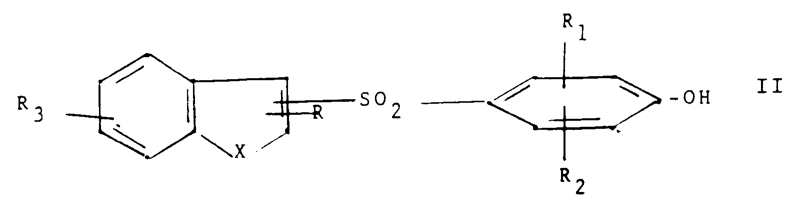 dans laquelle R, R₁, R₂, R₃ et X ont la même signification que dans la revendication 1, caractérisé en ce que l'on oxyde, dans un solvant, un dérivé hydroxy-4 benzènethio de formule générale : 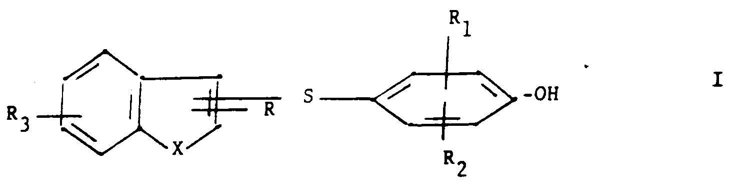 dans laquelle R, R₁, R₂, R₃ et X ont la même signification que dans la revendication 1, au moyen d'un agent d'oxydation pour obtenir les composés désirés.

9. Procédé de préparation de dérivés aminoalkoxybenzènesulfonyles de formule générale : 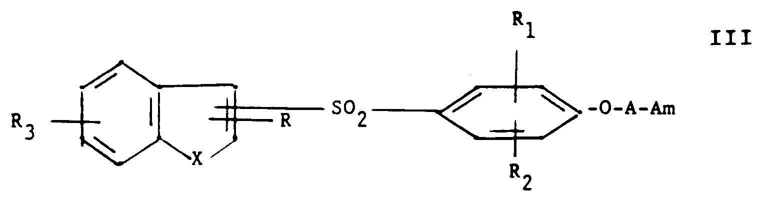 dans laquelle R, R₁, R₂, R₃ et X ont la même signification que dans la revendication 1, A représente un radical alkylène en C₂-C₅ ou hydroxy-2 propylène et Am représente un radical amino substitué, caractérisé en ce que :
(a) on oxyde, dans un solvant un dérivé hydroxy-4 benzènethio de formule générale : 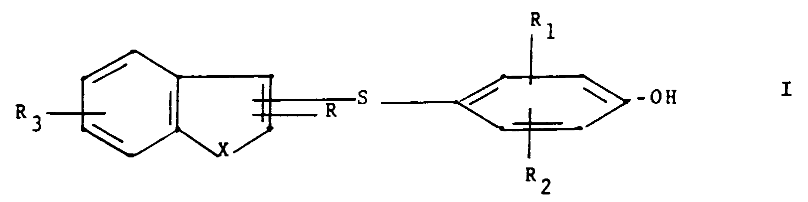 dans laquelle R, R₁, R₂, R₃ et X ont la même signification que dans la revendication 1, au moyen d'un agent d'oxydation pour obtenir un dérivé hydroxy-4 benzènesulfonyle de formule générale : 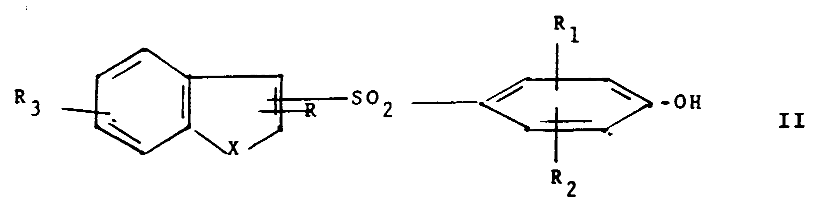 dans laquelle R, R₁, R₂, R₃ et X ont la même signification que dans la revendication 1,
(b) on condense en présence d'un agent basique le dérivé hydroxy-4 benzènesulfonyle ainsi formé avec un dihalogénoalkane de formule générale :
Hal - A - Hal VII
dans laquelle A a la même signification que précédemment et Hal représente un atome d'halogène et ce, au reflux, dans un solvant,
ou bien
(C₁) on condense le dérivé hydroxy-4 benzènesulfonyle ainsi formé avec un alcool halogéné de formule générale:
Hal - A - OH VIII
dans laquelle A et Hal ont la même signification que précédemment et ce , dans un solvant et en présence d'un agent basique, puis on condense le dérivé alcool obtenu avec un halogénure de formule générale :
Hal - W IX
dans laquelle Hal a la même signification que précédemment et W représente un radical alkylsulfonyle an C₁-C₄ ou arylsulfonyle en C₆-C₁₀, dans un solvant accepteur d'acide,
ou bien
(C₂) on chauffe le dérivé hydroxy-4 benzènesulfonyle ainsi formé au reflux avec une épihalohydrine en présence d'un agent basique et dans un solvant polaire, pour obtenir les dérivés sulfonyles hétérocycliques de formule générale : 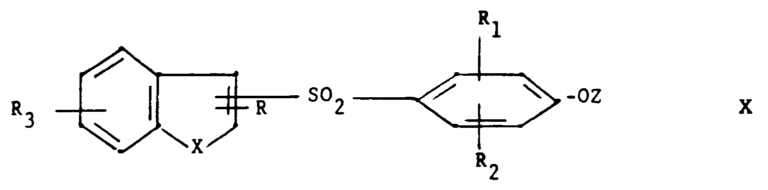 dans laquelle R, R₁, R₂, R₃ et X ont la même signification que précédemment et Z représente un radical de formule générale :
-A - Z₁
dans laquelle A a la même signification que précédemment et Z₁ représente un atome d'halogène, un radical alkylsulfonyloxy en C₁-C₄ ou arylsulfonyloxy en C₆-C₁₀, dérivé benzènesulfonyle de formule X que l'on fait réagir avec une amine de formule générale :
H - Am XI
dans laquelle Am a la même signification que précédemment, la réaction ayant lieu en présence d'un accepteur d'acide et dans un solvant ou encore en présence d'un excès d'amine de formule XI, pour obtenir les composés désirés sous forme de base libre que l'on peut faire réagir, si on le désire, avec un acide pour former un sel pharmaceutiquement acceptable de ce composé.

10. Procédé de préparation de dérivés aminoalkoxybenzènesulfonyles de formule générale : 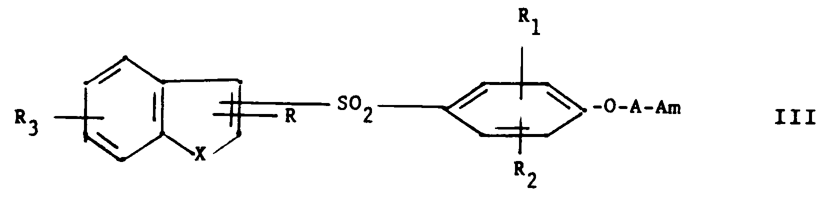 dans laquelle R, R₁, R₂, R₃ et X ont la même signification que dans la revendication 1, A représente un radical alkylène en C₂-C₅ et Am représente un radical amino substitué, caractérisé en ce que :
(a) on oxyde dans un solvant, un dérivé hydroxy-4 benzènethio de formule générale : 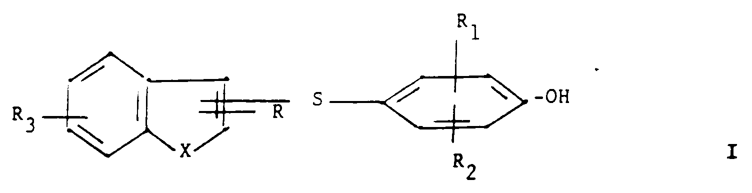 dans laquelle R, R₁, R₂, R₃ et X ont la même signification que dans la revendication 1, au moyen d'un agent d'oxydation pour obtenir un dérivé hydroxy-4 benzènesulfonyle de formule générale : 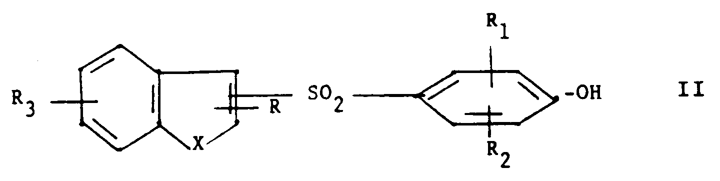 dans laquelle R, R₁, R₂, R₃ et X ont la même signification que dans la revendication 1,
(b) on traite le dérivé hydroxy-4 benzènesulfonyle ainsi formé, avec un halogènure de formule générale :
Hal - A - Am XII
dans laquelle A et Am ont la même signification que précédemment et Hal représente un atome d'halogène, la réaction étant conduite en présence d'un agent basique, pour obtenir les composés désirés sous forme de base libre que l'on peut, si on le désire, faire réagir avec un acide pour former un sel pharmaceutiquement acceptable de ce composé.

11. Procédé selon l'une des revendications 8 à 10, caractérisé en ce que l'agent d'oxydation est l'acide chloro-3 perbenzoïque ou le monoperphtalate de magnésium.

12. Procédé selon l'une des revendications 8 à 10, caractérisé en ce que l'on utilise de 2 à 2,5 équivalents d'agent d'oxydation par équivalent de dérivé benzènethio de formule I.

13. Procédé selon l'une des revendications 8 à 10, caractérisé en ce que l'oxydation a lieu à une température comprise entre -5°C et la température ambiante.

14. Procédé selon l'une des revendications 8 à 10, caractérisé en ce que l'oxydation a lieu dans un solvant polaire.

15. Procédé selon la revendication 14, caractérisé en ce que le solvant est le N, N-diméthylformamide, l'hexaméthylphosphotriamide, l'acétonitrile ou un alcool en C₁-C₄.

16. Procédé selon la revendication 9 ou 10, caractérisé en ce que Am représente :
- un radical de formule : 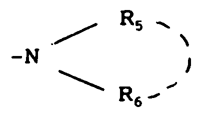 dans laquelle R₅ représente l'hydrogène ou un radical alkyle en C₁-C₈ et R₆ représente un radical alkyle en C₁-C₈ ou un radical de formule :
Alk - R₇
dans laquelle Alk représente une liaison simple ou un radical alkylène en C₁-C₅ et R₇ représente un radical pyridyle, phényle, méthylènedioxy-2,3 phényle, méthylènedioxy-3,4 phényle ou un groupement phényle substitué par un ou plusieurs substituants identiques ou différents sélectionnés parmi des atomes d'halogène, des groupements alkyles en C₁-C₄ ou des groupements alkoxy en C₁-C₄ ou R₅ et R₆, lorsqu'ils sont pris ensemble, représentent un radical alkylène ou alkénylène en C₃-C₆ éventuellement interrompu par -O-, -NH-, -N= ou 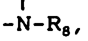 R₈ représentant un radical alkyle en C₁-C₄, phényle ou diphénylméthyle, ou
- un groupement de formule : 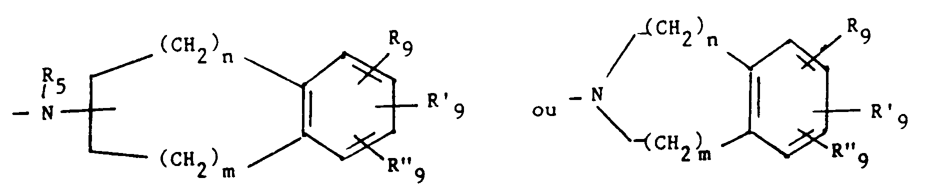 dans laquelle R₅ a la même signification que précédemment, R₉, R'₉ et R''₉, qui sont identiques ou différents, représentent chacun l'hydrogène, un atome d'halogène, un groupement alkyle en C₁-C₄ ou alkoxy en C₁-C₄ et n et m, identiques ou différents, représentent chacun 0, 1, 2 ou 3.

17. Procédé selon l'une des revendications 9 à 16, caractérisé en ce que l'on prépare le méthyl-1 isopropyl-3 [{[N-méthyl N-(diméthoxy-3, 4 β-phénéthyl)-amino]-3 propoxy}-4 benzènesulfonyl]-2 indole et ses sels pharmaceutiquement acceptables.

18. Procédé selon l'une des revendications 9 à 16, caractérisé en ce que l'on prépare le méthyl-1 isopropyl-3 [{[N-méthyl N-(diméthoxy-3,5 β-phénéthyl)-amino]-3 propoxy}-4 benzènesulfonyl]-2 indole et ses sels pharmaceutiquement acceptables.

19. Procédé selon l'une des revendications 9 à 16, caractérisé en ce que l'on prépare le [{[N-méthyl N-(diméthoxy-3,4 β-phénéthyl)amino]-3 propoxy}-4 benzènesulfonyl)-2 chloro-5 isopropyl-3 méthyl-1 indole et ses sels pharmaceutiquement acceptables.

20. Procédé selon la revendication 7, caractérisé en ce que le solvant est une solution aqueuse d'un alcool en C₁-C₄ ou d'un amide.
